# EUROPEAN PATENT APPLICATION

(11) **EP 4 592 287 A1**
(43) Date of publication of application: **30.07.2025**
(21) Application number: 23867205.9
(22) Date of filing: 23.08.2023
(51) Int. Cl.: C07D 417/12, C07D 413/12, A61K 31/496, A61P 15/10

(54) **2H-BENZOTRIAZOLE DERIVATIVE, PREPARATION METHOD THEREFOR, AND PHARMACEUTICAL COMPOSITION CONTAINING SAME**

(30) Priority: 19.09.2022 CN 202211135547
(71) Applicant: Orxes Therapeutics Co., Ltd., Shenyang, Liaoning 110136 (CN)
(72) Inventor: WANG, Peng, Shenyang, Liaoning 110136 (CN); ZHAO, Yuqing, Shenyang, Liaoning 110136 (CN); ZHOU, Yan, Shenyang, Liaoning 110136 (CN); ZHANG, Lirong, Shenyang, Liaoning 110136 (CN); ZHOU, Jie, Shenyang, Liaoning 110136 (CN); ZHOU, Xin, Shenyang, Liaoning 110136 (CN)
(74) Representative: Haseltine Lake Kempner LLP
(86) International application number: PCT/CN2023/114340
(87) International publication number: WO 2024/060912

(57) **Abstract**

The present invention provides a 2H-benzotriazole derivative, a preparation method thereof, and a pharmaceutical composition containing the same. It relates to the technical field of pharmacy, and particularly relates to a 2H-benzotriazole derivative as shown by formula (I), or a pharmaceutically acceptable salt thereof, or a pharmaceutical composition containing them, and use thereof in the manufacture of a medicament for treating and/or preventing premature ejaculation. According to the research for *in vivo* activity, the 2H-benzotriazole derivative of the present invention displays a significant anti-premature ejaculation effect, which is significantly higher than the on-sale drug dapoxetine, the only approved clinical drug, and has significant therapeutic characteristics and substantial technological advances. The 2H-benzotriazole derivative or its pharmaceutically acceptable salt of the present invention has great research and application prospects in serving as a medicament for treating premature ejaculation.

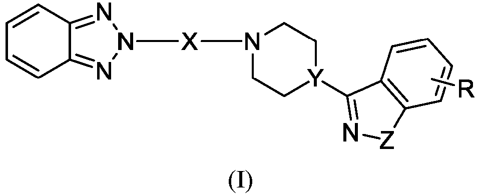

## Description

### Technical Field

The present invention relates to the technical field of pharmacy, and particularly relates to a novel 2H-benzotriazole derivative, a preparation method thereof, and a pharmaceutical composition containing the same.

### Background Art

Prospermia (Premature ejaculation) is one of the most common male sexual dysfunctions, with an incidence rate of 20% to 30%. The International Society for Sexual Medicine defines it as follows: premature ejaculation refers to the condition that ejaculation often or always occurs before or within about one minute after insertion of the vagina at the onset of initial sexual life (primary premature ejaculation); or the condition that the intra-vaginal ejaculation latency time (IELT) is significantly shortened, typically less than 3 minutes (secondary premature ejaculation); or the condition that ejaculation can never or almost never be controlled and delayed; with negative physical and psychological effects, such as distress, worry, depression, avoidance of sexual contact and the like. The causes of premature ejaculation are complicated, including disorders of central 5-hydroxytryptamine and dopamine system neurotransmitter, excessive sensitivity of penis head, genetic variation, psychological factors such as tension and/or anxiety, erectile dysfunction, chronic prostatitis, thyroid diseases, drugs, etc. Premature ejaculation is both psychological and physical disease, and according to the holistic concept of traditional Chinese medicine, this disease is a local symptom under maladjustment of the whole body. A new target for premature ejaculation treatment is psychological and physical co-adjustment, and overall and local co-treatment. The purpose of treatment is to improve the ejaculation control of the patient, improve the sexual life satisfaction of both spouses, and extend the ejaculation time. On the basis of explicit diagnosis and classification, the physician should discuss treatment expectations with the patient and his partner, and jointly formulate treatment goals and treatment regimen with them.

Dapoxetine hydrochloride is a selective 5-hydroxytryptamine reuptake inhibitor. It is the only drug in the world that is marketed as a therapeutic drug for premature ejaculation. Now it has been approved for premature ejaculation treatment as desired in more than fifty countries or regions including the European Union and China. Researches have shown that taking 30 mg or 60 mg of dapoxetine hydrochloride at the time of 1 to 2 hours before sexual intercourse is more effective than placebo, which increases IELT by 2.5 and 3.0 times respectively, improves ejaculation control, reduces pain of the patient, and improves sexual life satisfaction. The drug has similar curative effects in primary premature ejaculation and secondary premature ejaculation, and has treatment-related side effects which are dose-dependent, including nausea, diarrhea, headache, and dizziness. The initial dose of dapoxetine hydrochloride for treatment is recommended to be 30 mg. It is generally evaluated after using six times within four weeks, and in case the effect is poor, the dose can be increased to 60 mg. However, the clinical discontinuation rate is very high, and the cumulative discontinuation rate is increased over time, and reaches 90% or more after two years of initiation of treatment. The reasons for discontinuation include high cost (29.9%), disappointment with incurable premature ejaculation and the dependence of administration of the drug on demand (25.0%). Poor bioavailability of dapoxetine is an important reason for poor curative effect of dapoxetine and low patient satisfaction, resulting in a high clinical discontinuation rate. Therefore, it is of clinical significance to develop new drugs for treating premature ejaculation.

### Summary of the Invention

It is a primary object of the present invention to provide a 2H-benzotriazole derivative with medicinal value and a preparation method thereof, and a pharmaceutical composition containing the derivative.

It is a secondary object of the present invention to provide the use of the 2H-benzotriazole derivative and the pharmaceutical composition containing the derivative in the manufacture of medicaments for treating and/or preventing premature ejaculation.

The 2H-benzotriazole derivative disclosed in the present invention is a compound represented by formula (I) or a pharmaceutically acceptable salt of the compound, including a hydrochloride, a hydrobromide, a sulfate, a trifluoroacetate, a methanesulfonate, a tartrate, a malate, a citrate, and a succinate, and the salt may contain 0.5 to 3 molecules of crystal water: 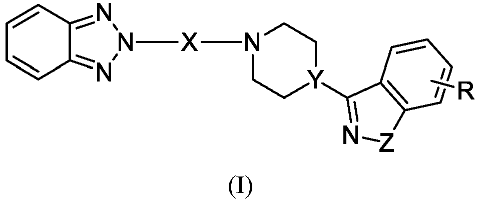 wherein
X represents: a C₃-C₄-alkyl or a C₃-C₄-alkoxy;
Y represents: CH or N;
Z represents: O or S;
R represents: optional substitution of hydrogen atom(s) by 1-3 halogen atoms selected from the group consisting of fluorine, chlorine, bromine.

Preferably, the 2H-benzotriazole derivative is any one of the following compounds:
A001: 1-(4-(4-benzoisoxazolyl) piperazin-1-yl) butyl-2H-benzotriazole
A002: 1-(3-(4-(3-(6-fluorobenzoisoxazolyl)) piperidin-1-yl) propyl-2H-benzotriazole
A003: 1-(4-(4-(3-(6-fluorobenzoisoxazolyl)) piperidin-1-yl) butyl-2H-benzotriazole
A004: 1-(3-(4-benzisothiazolyl) piperazin-1-yl) propyl-2H-benzotriazole
A005: 1-(3-(4-(3-(6-fluorobenzoisoxazolyl)) piperazin-1-yl) propyl-2H-benzotriazole
A006: 1-(4-(4-(3-(6-fluorobenzoisoxazolyl)) piperazin-1-yl) butyl-2H-benzotriazole

The specific chemical structural formulas are shown in the following table:

| No. | Chemical structure |
|---|---|
| A001 | 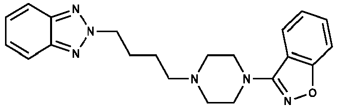 |
| A002 | 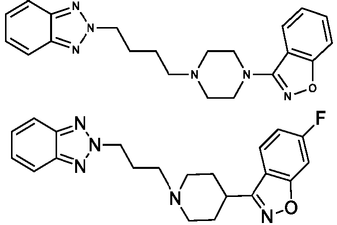 |
| A003 | 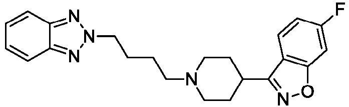 |
| A004 | 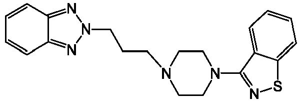 |
| A005 | 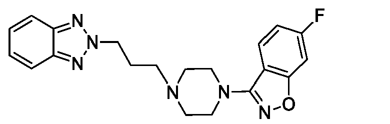 |
| A006 | 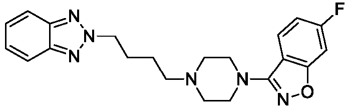 |

The general synthetic scheme is shown as follows: 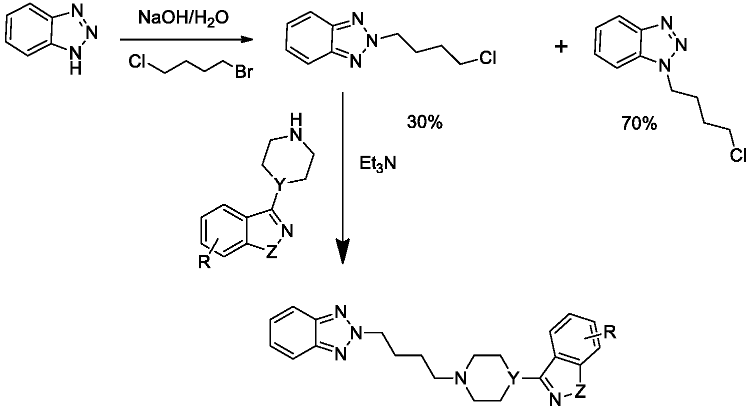 wherein X, Y, Z and R are defined as above.

Taking compound A001 as an example, substituted 1H-benzotriazole is used as raw material and reacted with 1-bromo-4-chlorobutane in an aqueous sodium hydroxide solution through substitution reaction to prepare 1-(4-chlorobutyl)-1H-benzotriazole (with a yield of about 70%) and 1-(4-chlorobutyl)-2H-benzotriazole (with a yield of about 30%). The obtained mixture is purified by silica gel column chromatography to give 1-(4-chlorobutyl)-2H-substituted benzotriazole (with a purity > 95%), which is then reacted with benzisoxazolpiperazine through condensation reaction to prepare compound A001. Compound A001 may finally be transformed to corresponding salt by acidification. Compounds A002-A006 and salts thereof can be prepared by the above-mentioned method.

Also provided is a pharmaceutical composition comprising any one or more of said 2H-benzotriazole derivatives, or a pharmaceutically acceptable salt thereof, and also a pharmaceutically acceptable carrier. The carrier refers to a conventional carrier in the field of pharmacy, including diluents; excipients such as water; binders such as cellulose derivatives, gelatin, polyvinylpyrrolidone; fillers such as starch; disintegrating agents such as calcium carbonate, sodium bicarbonate; lubricants such as calcium stearate or magnesium stearate. In addition, other adjuvants such as fragrances and sweeteners may also be added to the pharmaceutical composition. When used for oral administration, the pharmaceutical composition can be prepared as conventional solid preparations such as tablets, powders or capsules; when used for injection, it can be prepared as injection solution.

Various dosage forms of the pharmaceutical composition according to the present invention are prepared according to conventional methods in the art, wherein the content of the active ingredient is 0.1% to 99.5% (by weight).

Also provided is use of the 2H-benzotriazole derivative, or its pharmaceutically acceptable salt, or the pharmaceutical composition containing the derivative according to the present invention in the manufacture of medicaments for treating and/or preventing premature ejaculation.

### Beneficial effects of the present invention:

The 2H-benzotriazole derivative of the present invention displays a significant effect of inhibiting premature ejaculation, and has significant therapeutic characteristics and substantial technological advances compared with dapoxetine, the only approved clinical drug. The therapeutic characteristics and advantages thereof are as follows:
The 2H-benzotriazole derivative or its pharmaceutically acceptable salt of the present invention is a group of 2H-benzotriazole derivatives with new structures. According to research by experiments *in vivo,* the effect of inhibiting premature ejaculation of such compound is significantly higher than that of the commercially available drug dapoxetine. The 2H-benzotriazole derivative or its pharmaceutically acceptable salt of the present invention has great research and application prospects in serving as a medicament for treating premature ejaculation.

### Brief Description of Drawings

Figure 1: Histogram of the total number of ejaculations of Wistar rats within 30 min after PCA induction, One-way ANOVA, Dunnett post hoc, ***P<0.001, compared with PE model group, N=5.
Figure 2: Histogram of the initial ejaculation latency period of Wistar rats within 30 min after PCA induction, One-way ANOVA, Dunnett post hoc, *P<0.05, **P<0.01, ***P<0.001, compared with PE model group, N=5.
Figure 3: Histogram of the number of seminal vesicle contractions of Wistar rats within 30 min after PCA induction, One-way ANOVA, Dunnett post hoc, ***P<0.001, compared with PE model group, N=5.
Figure 4: Histogram of the latency period of initial seminal vesicle contraction in Wistar rats within 30 min after PCA induction, One-way ANOVA, Dunnett post hoc, **P<0.01, ***P<0.001, compared with PE model group, N=5.
Figure 5: Histogram of seminal vesicle basal pressure of Wistar rats within 30 min after PCA induction, One-way ANOVA, Dunnett post hoc, ***P<0.001, compared with PE model group, N=5.
Figure 6: Histogram of seminal vesicle peak pressure of Wistar rats within 30 min after PCA induction, One-way ANOVA, Dunnett post hoc, ***P<0.001, compared with PE model group, N=5.

### Detailed Description of the Invention

Reagents and solvents were purchased from Sigma-Aldrich or Fisher Scientific and used without further purification. All reactions were subjected to thin layer chromatography (silica gel GF-254 thin layer plate) and monitored by LC-MS. Column chromatography purification was conducted on 300-400 mesh silica gel (Qingdao Ocean Chemical Co. Ltd.). ¹H and ¹³C NMR spectra were obtained through a Bruker AV-400 nuclear magnetic resonance instrument, with TMS as an internal standard. The purity of the compound determined by LC-MS analysis was greater than 95%. LC-MS analysis was also used to record the MS spectrum of the compound and was performed using Shimadzu LCMS-2020.

### General synthetic process:

### (1) Preparation of key intermediate: 1-(4-chlorobutyl) -2H-benzotriazole (A1)

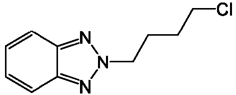

30.0 g (251 mmol) of benzotriazole, 39.3 g (229 mmol) of 1-bromo-4-chlorobutane, 1.85 g (6 mmol) of tetrabutylammonium bromide, and 240 g (20%) of sodium hydroxide aqueous solution were placed in a 500 mL single-necked flask and stirred thoroughly until the material was completely dissolved. The temperature was raised to 60°C, and the reaction was performed with stirring for 2 h and monitored by TLC. After the reaction was completed, the reaction mixture was extracted with dichloromethane (240 mL×3), dried over anhydrous sodium sulfate and then distilled off the solvent under reduced pressure. Purification via silica gel column chromatography gave 10.23 g of light yellow oily liquid (with a yield of 19.44%).

### (2) Preparation of key intermediate: 1-(3-chloropropyl)-2H-benzotriazole (A2)

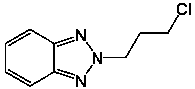

36.0 g (302 mmol) of benzotriazole, 47.5 g (302 mmol) of 1-bromo-3-chlorobutane, 2.22 g (6.8 mmol) of tetrabutylammonium bromide, and 240 g (20%) of sodium hydroxide aqueous solution were placed in a 500 mL single-necked flask and stirred thoroughly until the material was completely dissolved. The temperature was raised to 60°C, and the reaction was performed with stirring for 2 h and monitored by TLC. After the reaction was completed, the reaction mixture was extracted with dichloromethane (240 mL×3), dried over anhydrous sodium sulfate and then distilled off the solvent under reduced pressure. Purification via silica gel column chromatography gave 11.71 g of light yellow oily liquid (with a yield of 19.82%).

### (3) Preparation of 6-fluoro-3-(piperazin-1-yl) benzoisoxazole (A3)

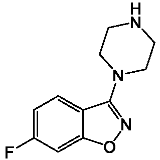

1.58 g (10 mmol) of 2-chloro-4-fluorobenzaldehyde, 0.84 g (10 mmol) of hydroxylamine hydrochloride, 1.64 g (20 mmol) of sodium acetate, 30 mL ethanol and 10 mL water were placed in a 100 mL single-necked flask for reaction for 2 h. The reaction was monitored by TLC. After the reaction was completed, the ethanol was removed by reduced pressure distillation, filtered by suction, and the filter cake was washed with purified water to obtain 1.65 g of (E)-2-chloro-4-fluoro-benzaldehyde oxime (with a yield of 95%). 1.73 g (10 mmol) of (E)-2-chloro-4-fluorobenzaldehyde oxime and 15 mL of N,N-dimethylformamide were placed in a 50 mL single-necked flask, and 1.47 g (11 mmol) of N-chlorosuccinimide was added. The reaction was performed at room temperature for 1 h and monitored by TLC. After the reaction was completed, the reaction solution was dripped into 300 mL of purified water and stirred. After suction filtration, 1.85 g of (Z)-2-chloro-4-fluoro-chlorohydroxyimine benzyl was obtained (with a yield of 89%). 1.04 g (5 mmol) of (Z)-2-chloro-4-fluoro-chlorohydroxyimine benzyl, 3.44 g (40 mmol) of anhydrous piperazine, and 1.01 g (10 mmol) of trimethylamine were dissolved in 30 mL of dichloromethane in a 100 mL single-necked flask. The reaction was performed for 2 h and monitored by TLC. After the reaction was completed, a saturated copper sulfate solution was added to the reaction solution under stirring until no blue flocculent precipitate was produced. The reaction solution was filtered by suction, washed with saturated saline solution, extracted with dichloromethane, and distilled off the solvent to obtain 0.79 g of (Z)-(2-chloro-4-fluorophenyl)-1-piperazinyl methanone oxime (with a yield of 61%). 0.65 g (5 mmol) of (Z)-(2-chloro-4-fluorophenyl)-1-piperazinyl methanone oxime, 0.56 g (5 mmol) of potassium tert-butoxide, and 10 mL of 1,4-dioxane were placed in a 50 mL single-necked flask, and reacted at 100°C for 12 h. The reaction was monitored by TLC. After the reaction was completed, the solvent was distilled off under reduced pressure to obtain a yellow oil. Separation and purification via silica gel column chromatography gave 0.282 mg of 6-fluoro-3-(piperazin-1-yl) benzoisoxazole as a white powdery solid (with a yield of 51%).

### (4) General preparation method of the target compound (taking A004 as an example)

1.88 g of 3-(1-piperazinyl)-1,2-benzisothiazole, triethylamine (3.3 g), potassium iodide (1.4 g), and 15 mL of acetonitrile were added to a 50 mL single-necked flask and stirred thoroughly until the raw material was completely dissolved. The intermediate A2 (1.8 g) was added and the reaction was heated to 81°C under reflux for 18 h. After the reaction was completed, as determined by TLC, the reaction solution was cooled to room temperature, filtered by suction, and the filtrate was distilled under reduced pressure to remover the solvent to obtain a yellow oil. The residue was washed with saturated saline solution, extracted with dichloromethane, and distilled under reduced pressure to remove the solvent to obtain an oil. The oil was dissolved in anhydrous ethanol, and adjusted to pH=1 by adding an solution of hydrochloric acid dissolved in ethanol. After the completion of dripping, it was stirred at room temperature for 1 hour to precipitate solid, which was filtered off. The filter cake was recrystallized with anhydrous ethanol to give the final product.

### Example 1

### Preparation of 1-(4-(4-benzoisoxazolyl) piperazin-1-yl) butyl-2H-benzotriazole

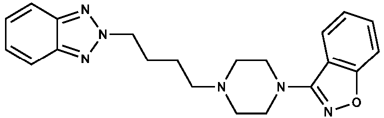

The synthesis followed the general preparation method. Recrystallization with anhydrous ethanol gave 1.12 g of a white powdery solid (with a yield of 42%). ¹H NMR (300 MHz, DMSO-*d*₆) *δ* 8.04 - 7.85 (m, 3H), 7.58 (d, *J =* 3.9 Hz, 2H), 7.51 - 7.40 (m, 2H), 7.29 (dt, *J =* 8.0, 4.0 Hz, 1H), 4.80 (t, *J =* 6.9 Hz, 2H), 3.46 (t, *J =* 4.9 Hz, 4H), 2.51 (dq, *J =* 5.6, 3.5, 2.6 Hz, 4H), 2.37 (t, *J =* 7.1 Hz, 2H), 2.17 - 1.98 (m, 2H), 1.47 (p, *J =* 7.3 Hz, 2H); ¹³C NMR (100 MHz, DMSO-*d*₆) *δ* 163.64, 161.29, 144.10, 130.39, 126.68, 123.42, 123.10, 118.24, 116.04, 110.53, 57.44, 56.32, 52.48, 48.15, 27.84, 23.57; HR-MS (ESI) *m*/*z*: calcd for C₂₁H₂₅N₆O [M+H]⁺ 377.2084 found 377.2089.

### Example 2

### Preparation of 1-(3-(4-(3-(6-fluorobenzoisoxazolyl)) piperidin-1-yl) propyl-2H-benzotriazole hydrochloride

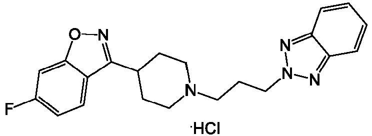

The synthesis followed the general preparation method. Recrystallization with ethyl acetate gave 1.36 g of a white powdery solid (with a yield of 42%). ¹H NMR (400 MHz, DMSO-*d*₆) δ 11.16 (s, 1H), 8.22 (dd, *J =* 8.8, 5.2 Hz, 1H), 7.94 (dd, *J =* 6.5, 3.1 Hz, 2H), 7.71 (dd, *J =* 9.1, 2.2 Hz, 1H), 7.46 (dd, *J =* 6.5, 3.1 Hz, 2H), 7.32 (td, *J =* 9.1, 2.2 Hz, 1H), 4.93 (t, *J =* 6.8 Hz, 2H), 3.55 - 3.43 (m, 1H), 3.30 - 2.95 (m, 5H), 2.74 - 2.58 (m, 2H), 2.40 (dd, *J =* 13.3, 3.5 Hz, 1H), 2.29 - 2.11 (m, 2H). ¹³C NMR (100 MHz, DMSO-*d*₆) δ 165.43, 163.78, 163.64, 162.97, 160.54, 144.23, 126.97, 124.39, 124.28, 118.32, 117.13, 113.34, 113.09, 98.12, 97.85, 53.92, 53.87, 51.84, 31.60, 27.31, 24.31. LC-MS (ESI) m/z: 380.17 [M+1]⁺.

### Example 3

### Preparation of 1-(4-(4-(3-(6-fluorobenzoisoxazolyl)) piperidin-1-yl) butyl-2H-benzotriazole hydrochloride

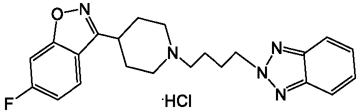

The synthesis followed the general preparation method. Recrystallization with anhydrous ethanol gave 1.72 g of a white powdery solid (with a yield of 51%). ¹H NMR (400 MHz, DMSO-*d*₆) δ 11.01 (s, 1H), 8.24 (dd, *J =* 8.8, 5.3 Hz, 1H), 7.99 - 7.87 (m, 2H), 7.71 (dd, *J* = 9.1, 2.2 Hz, 1H), 7.44 (dp, *J =* 5.9, 2.9 Hz, 2H), 7.33 (td, *J =* 9.1, 2.2 Hz, 1H), 4.83 (t, *J =* 6.8 Hz, 2H), 3.57 (d, *J =* 12.0 Hz, 2H), 3.52 - 3.42 (m, 1H), 3.23 - 3.02 (m, 4H), 2.40 (qd, *J =* 13.2, 3.8 Hz, 2H), 2.29 - 2.03 (m, 4H), 1.90 - 1.69 (m, 2H). ¹³C NMR (100 MHz, DMSO-*d*₆) δ 165.44, 163.78, 163.64, 162.97, 160.58, 144.17, 126.82, 124.38 (d, *J =* 11.2 Hz), 118.27 (d, *J* = 3.2 Hz), 117.14, 113.33, 113.08, 98.12, 97.85, 55.76 (d, *J =* 13.5 Hz), 51.70, 48.98, 31.65, 27.19 (d, *J =* 7.3 Hz), 24.54, 20.93. LC-MS (ESI) m/z: 394.21 [M+1]⁺.

### Example 4

### Preparation of 1-(3-(4-benzisothiazolyl) piperazin-1-yl) propyl-2H-benzotriazole hydrochloride

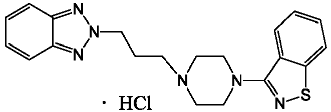

The synthesis followed the general preparation method. Recrystallization with anhydrous ethanol gave 1.21 g of a white powdery solid (with a yield of 44%). ¹H NMR (300 MHz, DMSO-*d*₆) *δ* 11.66 (s, 1H), 8.17 - 8.05 (m, 3H), 8.01 (d, *J =* 8.4 Hz, 1H), 7.67 - 7.55 (m, 2H), 7.45 (q, *J =* 7.7 Hz, 2H), 4.91 (t, *J =* 6.9 Hz, 2H), 4.05 (d, *J =* 13.6 Hz, 2H), 3.58 (q, *J =* 12.9, 11.8 Hz, 4H), 3.29 (d, *J =* 10.3 Hz, 4H), 2.67 - 2.33 (m, 2H); ¹³C NMR (100 MHz, DMSO-*d*₆) *δ* 162.65, 152.58, 145.66, 133.28, 128.58, 127.80, 127.43, 125.08, 124.53, 124.47, 121.66, 119.64, 111.14, 53.59, 51.09, 46.80, 45.50, 24.17; HR-MS (ESI) *m*/*z*: calcd for C₂₀H₂₃N₆S [M+H]⁺ 379.1699 found 379.1701.

### Example 5

### Preparation of 1-(3-(4-(3-(6-fluorobenzoisoxazolyl)) piperazin-1-yl) propyl-2H-benzotriazole hydrochloride

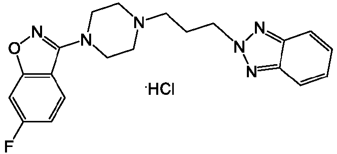

The synthesis followed the general preparation method. Recrystallization with anhydrous ethanol gave 165 mg of a white powdery solid (with a yield of 51%). ¹H NMR (400 MHz, DMSO-*d*₆) δ 11.67 (s, 1H), 8.09 (dd, *J =* 8.9, 5.2 Hz, 1H), 7.94 (dd, *J =* 6.6, 3.1 Hz, 2H), 7.59 (dd, *J =* 9.1, 2.3 Hz, 1H), 7.46 (dd, *J =* 6.6, 3.1 Hz, 2H), 7.24 (td, *J =* 9.1, 2.3 Hz, 1H), 4.92 (t, *J* = 6.8 Hz, 2H), 4.09 (d, *J =* 13.5 Hz, 2H), 3.61 (d, *J =* 12.2 Hz, 4H), 3.26 (t, *J =* 8.2 Hz, 4H), 2.66 - 2.54 (m, 2H). ¹³C NMR (100 MHz, DMSO-*d*₆) δ 165.22, 164.69, 164.55, 162.75, 160.25, 144.23, 126.95, 124.85, 124.74, 118.33, 112.49, 112.40, 112.16, 98.26, 97.99, 53.89, 53.51, 50.47, 45.11, 24.14. LC-MS (ESI) m/z: 381.15 [M+1]⁺.

### Example 6

### Preparation of 1-(4-(4-(3-(6-fluorobenzoisoxazolyl)) piperazin-1-yl) butyl-2H-benzotriazole hydrochloride

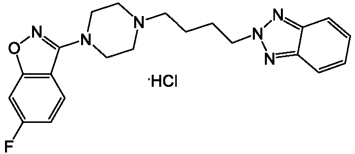

The synthesis followed the general preparation method. Recrystallization with anhydrous ethanol gave 182 mg of a white powdery solid (with a yield of 54%). ¹H NMR (400 MHz, DMSO-*d*₆) δ 11.29 (s, 1H), 8.09 (dd, *J =* 8.9, 5.2 Hz, 1H), 7.93 (dd, *J =* 6.5, 3.1 Hz, 2H), 7.60 (dd, *J =* 9.1, 2.3 Hz, 1H), 7.51 - 7.40 (m, 2H), 7.24 (td, *J =* 9.1, 2.3 Hz, 1H), 4.82 (t, *J =* 6.8 Hz, 2H), 4.08 (d, *J =* 13.2 Hz, 2H), 3.54 (d, *J =* 12.5 Hz, 4H), 3.21 (t, *J =* 8.2 Hz, 4H), 2.12 (t, *J =* 7.5 Hz, 2H), 1.79 (dd, *J =* 8.0, 3.6 Hz, 2H). ¹³C NMR (100 MHz, DMSO-*d*₆) δ 166.82 - 162.55 (m), 160.28, 144.16, 126.84, 124.85, 124.74, 118.29, 112.49, 112.18, 98.29, 98.02, 55.80, 55.35, 50.46, 45.10, 27.06, 20.87. LC-MS (ESI) m/z: 395.18 [M+1]⁺.

### Example 7

Research of *in vivo* activity of compounds A001-A006 on premature ejaculation model

### 7.1 Materials and methods

### 7.1.1 Compound information

| Compound name | Traits | Purity (%) | Source | Batch No. |
|---|---|---|---|---|
| A001 | Powder | 99 | Self-prepared | NA |
| A002 | Powder | 99 | Self-prepared | NA |
| A003 | Powder | 99 | Self-prepared | NA |
| A004 | Powder | 99 | Self-prepared | NA |
| A005 | Powder | 99 | Self-prepared | NA |
| A006 | Powder | 99 | Self-prepared | NA |
| Dapoxetine hydrochloride | Powder | 100 | Shaanxi New Drug | T0039 |
| P-chloramphetamine hydrochloride (PCA) | Powder | 95 | Beijing Lambert | LMFB022 |

### 7.2 Experimental scheme

### 7.2.1 Animal information

| | |
|---|---|
| Variety: | Rat |
| Strain: | Wistar |
| Gender: | Male, randomly selected |
| Weight: | ≈300 g |
| Supplier: | Beijing Vital River Laboratory Animal Technology Co. Ltd |
| Level: | SPF |
| Number of animals: | 105 |
| Adaptation period: | About 7 days |

### 7.2.2 Animal adaptation:

After the animals arrived at the animal facility, they were fed adaptively for at least one week. During this period, animal health and whether there were any physiological and behavioral abnormalities were monitored and all abnormal animals were removed from the research.

### 7.2.3 Feeding environment

The environment of the animal house was controlled at a temperature of 18-26°C and a humidity of 30-70%, and gave a 12 h light/12 h dark cycle. The 12 h dark cycle may be temporarily interrupted to accommodate to the research proposal.

### 7.2.4 Food and water

Rat maintenance feeds (provided by Jiangsu Xietong Pharmaceutical Bio-Engineering Co. Ltd.) and reverse osmosis water were available at any time during the research.

### 7.2.5 Animal selection and fasting

The animals used in this research were selected based on the health condition of the animals and adaptability to the cage culture. Before the experiment, the animals had no fasting or water deprivation.

### 7.2.6 Animal grouping

The animals were weighed before the experiment, and randomly grouped according to the weight, specifically as follows:

| Time to start grouping | | The day before the experiment | |
|---|---|---|---|
| Number of animals per cage | | 5 | |
| Grouping requirements | | Random grouping was performed according to the animal weight. | |
| Group | Number of animals per group | Dose | Administration route |
| 1-Sham group | 5 | NA | NA |
| 2- PE model group | 5 | Solvent + PCA 5 mg/kg | p.o.+ i.p. |
| 3-Dapoxetine group | 5 | Dapoxetine 2 mg/kg + PCA 5 mg/kg | i.v.+ i.p. |
| 4-A001 low dose group | 5 | A001, 0.125 mg/kg + PCA 5 mg/kg | p.o.+ i.p. |
| 5-A001 medium dose group | 5 | A001, 0.5 mg/kg + PCA 5 mg/kg | p.o.+ i.p. |
| 6-A001 high dose group | 5 | A001, 2.0 mg/kg + PCA 5 mg/kg | p.o.+ i.p. |
| 7-A002 low dose group | 5 | A002, 0.125 mg/kg + PCA 5 mg/kg | p.o.+ i.p. |
| 8-A002 medium dose group | 5 | A002, 0.5 mg/kg + PCA 5 mg/kg | p.o.+ i.p. |
| 9-A002 high dose group | 5 | A002, 2.0 mg/kg + PCA 5 mg/kg | p.o.+ i.p. |
| 10-A003 low dose group | 5 | A003, 0.125 mg/kg + PCA 5 mg/kg | p.o.+ i.p. |
| 11-A003 medium dose group | 5 | A003, 0.5 mg/kg + PCA 5 mg/kg | p.o.+ i.p. |
| 12-A003 high dose group | 5 | A003, 2.0 mg/kg + PCA 5 mg/kg | p.o.+ i.p. |
| 13-A004 low dose group | 5 | A004, 0.125 mg/kg + PCA 5 mg/kg | p.o.+ i.p. |
| 14-A004 medium dose group | 5 | A004, 0.5 mg/kg + PCA 5 mg/kg | p.o.+ i.p. |
| 15-A004 high dose group | 5 | A004, 2.0 mg/kg + PCA 5 mg/kg | p.o.+ i.p. |
| 16-A005 low dose group | 5 | A005, 0.125 mg/kg + PCA 5 mg/kg | p.o.+ i.p. |
| 17-A005 medium dose group | 5 | A005, 0.5 mg/kg + PCA 5 mg/kg | p.o.+ i.p. |
| 18-A005 high dose group | 5 | A005, 2.0 mg/kg + PCA 5 mg/kg | p.o.+ i.p. |
| 19-A006 low dose group | 5 | A006, 0.125 mg/kg + PCA 5 mg/kg | p.o.+ i.p. |
| 20-A006 medium dose group | 5 | A006, 0.5 mg/kg + PCA 5 mg/kg | p.o.+ i.p. |
| 21-A006 high dose group | 5 | A006, 2.0 mg/kg + PCA 5 mg/kg | p.o.+ i.p. |

| | | | |
|---|---|---|---|
| Note: p.o. denotes oral administration; i.p. denotes intraperitoneal injection; PCA denotes p-chloramphetamine hydrochloride. | | | |

### 7.2.7 Experimental procedure:

Male Wistar rats with a weight of about 300 g were adapted for one week before the experiment was started. On the day of the experiment, the animals were anesthetized with a combination of 20 mg/kg of Zoletil 50 via i.p. and 8 mg/kg of xylazine via i.p., and the body temperature was maintained at 37°C with a thermal insulation blanket. Exposed common carotid artery was intubated with PE50 to monitor real-time arterial pressure (i.e. systolic pressure, diastolic pressure, and mean arterial pressure). The seminal vesicles and cavernosal muscles of the animals were exposed. The stable baselines of the pressure of the seminal vesicle and the cavernosal myoelectricity were recorded for 10 min. Then the drug was administered through the tail vein for the dapoxetine group, and the tested compound was orally administered for the tested compound group (for the PE model group, the drug was replaced with a solvent). After 1 min, for inducing PE modeling, 5 mg/kg of PCA was injected intraperitoneally, and then the seminal vesicle pressure curve and the change of bulbospongiosus EMG were continuously recorded for 30 min.

### Experimental endpoint:

A. Within 30 min, the actual number of ejaculations of the animals and the latency period of the first ejaculation;
B. Within 30 min, the number of the seminal vesicle contractions, the latency period of the first contraction, the basal pressure and the peak pressure.

### 7.2.8 Data Analysis

The experimental data were expressed by means±S.E.M., and statistical analysis was performed by GraphPad Prism 7.0 software. Single factor variance analysis (One-way ANOVA) was used for comparison between multiple groups, and Dunnett't-test was used for statistical analysis in Post hoc test. Student's t-test was used for comparison between two groups. P < 0.05 was considered as a significant difference.

### 7.3 Results

**Table 1 Original data list of various premature ejaculation indexes of Wistar rats within 30 min after PCA induction**

| | Actual number of ejaculations | The first ejaculation latency period (s) | Number of the seminal vesicle contractions | The first contraction latency period (s) | Seminal vesicle basal pressure (mmHg) | Seminal vesicle peak pressure (mmHg) |
|---|---|---|---|---|---|---|
| Sham group | | | | | | |
| **1** | 0 | 1800 | 1 | 1605 | 4.45 | 4.85 |
| **2** | 0 | 1800 | 1 | 1782 | 3.77 | 4.28 |
| **3** | 0 | 1800 | 0 | 1800 | 3.89 | 4.36 |
| **4** | 0 | 1800 | 0 | 1800 | 3.97 | 4.07 |
| **5** | 0 | 1800 | 0 | 1800 | 4.66 | 4.91 |

| PE model group | | | | | | |
|---|---|---|---|---|---|---|
| **6** | 16 | 870 | 19 | 784 | 7.78 | 39.15 |
| **7** | 12 | 843 | 17 | 788 | 8.15 | 37.07 |
| **8** | 25 | 649 | 29 | 605 | 8.49 | 38.44 |
| **9** | 21 | 558 | 26 | 517 | 7.91 | 40.19 |
| **10** | 16 | 915 | 21 | 839 | 8.3 | 40.76 |

| Dapoxetine group | | | | | | |
|---|---|---|---|---|---|---|
| **11** | 1 | 1558 | 1 | 1448 | 5.16 | 6.89 |
| **12** | 0 | 1800 | 1 | 1705 | 4.97 | 8.1 |
| **13** | 0 | 1800 | 0 | 1800 | 5.37 | 6.46 |
| **14** | 1 | 1256 | 1 | 1749 | 5.88 | 6.78 |
| **15** | 2 | 967 | 3 | 866 | 4.96 | 7.03 |

| A001, 0.125 mg/kg group | | | | | | |
|---|---|---|---|---|---|---|
| **16** | 8 | 996 | 10 | 784 | 6.69 | 17.17 |
| **17** | 9 | 726 | 11 | 788 | 7.04 | 21.45 |
| **18** | 11 | 766 | 12 | 605 | 7.28 | 22.67 |
| **19** | 10 | 823 | 13 | 517 | 6.91 | 23.39 |
| **20** | 11 | 969 | 16 | 839 | 7.78 | 20.58 |

| A001, 0.5 mg/kg group | | | | | | |
|---|---|---|---|---|---|---|
| **21** | 0 | 1800 | 0 | 1800 | 3.88 | 4.89 |
| **22** | 0 | 1800 | 0 | 1800 | 4.19 | 5.09 |
| **23** | 2 | 1399 | 5 | 1298 | 5.77 | 11.06 |
| **24** | 3 | 1311 | 4 | 1147 | 5.08 | 16.57 |
| **25** | 2 | 1291 | 6 | 1125 | 5.68 | 14.89 |

| A001, 2 mg/kg group | | | | | | |
|---|---|---|---|---|---|---|
| **26** | 2 | 1055 | 4 | 968 | 6.88 | 18.65 |
| **27** | 0 | 1800 | 0 | 1800 | 4.68 | 6.99 |
| **28** | 0 | 1800 | 1 | 1687 | 4.38 | 6.07 |
| **29** | 0 | 1800 | 1 | 1774 | 4.92 | 7.32 |
| **30** | 0 | 1800 | 1 | 1629 | 3.98 | 6.05 |

| A002, 0.125 mg/kg group | | | | | | |
|---|---|---|---|---|---|---|
| **31** | 2 | 1422 | 5 | 1266 | 5.88 | 12.33 |
| **32** | 6 | 933 | 6 | 833 | 6.10 | 16.17 |
| **33** | 3 | 966 | 6 | 825 | 6.02 | 18.07 |
| **34** | 2 | 1276 | 5 | 1198 | 4.66 | 12.06 |
| **35** | 5 | 1345 | 3 | 1499 | 3.97 | 9.88 |

| A002, 0.5 mg/kg group | | | | | | |
|---|---|---|---|---|---|---|
| **36** | 2 | 1233 | 3 | 1176 | 5.76 | 12.54 |
| **37** | 3 | 1346 | 4 | 1307 | 5.88 | 11.66 |
| **38** | 1 | 1432 | 2 | 1355 | 5.79 | 7.05 |
| **39** | 0 | 1800 | 0 | 1800 | 3.67 | 4.27 |
| **40** | 0 | 1800 | 0 | 1800 | 3.87 | 5.11 |

| A002, 2 mg/kg group | | | | | | |
|---|---|---|---|---|---|---|
| **41** | 0 | 1800 | 2 | 1568 | 4.15 | 8.87 |
| **42** | 0 | 1800 | 0 | 1800 | 4.12 | 6.08 |
| **43** | 0 | 1800 | 0 | 1800 | 4.85 | 5.09 |
| **44** | 0 | 1800 | 0 | 1800 | 4.11 | 4.77 |
| **45** | 0 | 1800 | 0 | 1800 | 3.93 | 5.03 |

| A003, 0.125 mg/kg group | | | | | | |
|---|---|---|---|---|---|---|
| **46** | 0 | 1800 | 3 | 1458 | 3.97 | 4.08 |
| **47** | 0 | 1800 | 3 | 1525 | 3.78 | 4.25 |
| **48** | 4 | 865 | 8 | 707 | 5.98 | 16.58 |
| **49** | 5 | 778 | 8 | 658 | 5.59 | 14.17 |
| **50** | 4 | 806 | 7 | 735 | 5.17 | 18.06 |

| A003, 0.5 mg/kg group | | | | | | |
|---|---|---|---|---|---|---|
| **51** | 6 | 845 | 9 | 778 | 5.88 | 18.97 |
| **52** | 6 | 903 | 9 | 814 | 6.03 | 20.11 |
| **53** | 2 | 1175 | 5 | 1009 | 5.19 | 12.84 |
| **54** | 0 | 1800 | 0 | 1800 | 3.9 | 4.09 |
| **55** | 0 | 1800 | 0 | 1800 | 4.2 | 4.76 |

| A003, 2 mg/kg group | | | | | | |
|---|---|---|---|---|---|---|
| **56** | 0 | 1800 | 1 | 1623 | 4.65 | 8.13 |
| **57** | 0 | 1800 | 0 | 1800 | 4.17 | 5.24 |
| **58** | 0 | 1800 | 0 | 1800 | 4.55 | 4.9 |
| **59** | 0 | 1800 | 0 | 1800 | 4.62 | 4.89 |
| **60** | 0 | 1800 | 0 | 1800 | 3.92 | 4.86 |

| A004, 0.125 mg/kg group | | | | | | |
|---|---|---|---|---|---|---|
| **61** | 0 | 1800 | 3 | 1400 | 4.09 | 4.21 |
| **62** | 3 | 865 | 4 | 797 | 5.98 | 13.38 |
| **63** | 6 | 678 | 8 | 757 | 5.59 | 16.13 |
| **64** | 4 | 806 | 6 | 735 | 5.17 | 15.05 |
| **65** | 0 | 1800 | 2 | 1525 | 4.18 | 4.25 |
| | | | | | | |

| A004, 0.5 mg/kg group | | | | | | |
|---|---|---|---|---|---|---|
| **66** | 5 | 845 | 7 | 798 | 5.89 | 17.95 |
| **67** | 6 | 903 | 8 | 819 | 6.07 | 29.19 |
| **68** | 2 | 1175 | 3 | 1009 | 5.18 | 13.88 |
| **69** | 0 | 1800 | 0 | 1800 | 3.99 | 4.79 |
| **70** | 0 | 1800 | 0 | 1800 | 4.32 | 4.77 |

| A004, 2 mg/kg group | | | | | | |
|---|---|---|---|---|---|---|
| **71** | 0 | 1800 | 0 | 1800 | 4.68 | 5.18 |
| **72** | 0 | 1800 | 0 | 1800 | 4.67 | 5.26 |
| **73** | 0 | 1800 | 0 | 1800 | 4.65 | 4.96 |
| **74** | 0 | 1800 | 0 | 1800 | 4.66 | 4.79 |
| **75** | 0 | 1800 | 0 | 1800 | 3.97 | 4.87 |

| A005, 0.125 mg/kg group | | | | | | |
|---|---|---|---|---|---|---|
| **76** | 0 | 1800 | 2 | 1458 | 3.99 | 4.09 |
| **77** | 0 | 1800 | 3 | 1525 | 3.98 | 4.29 |
| **78** | 4 | 965 | 6 | 707 | 5.98 | 16.56 |
| **79** | 4 | 878 | 8 | 658 | 5.59 | 17.17 |
| **80** | 5 | 806 | 7 | 738 | 5.18 | 18.08 |

| A005, 0.5 mg/kg group | | | | | | |
|---|---|---|---|---|---|---|
| **81** | 5 | 845 | 6 | 778 | 5.89 | 18.99 |
| **82** | 5 | 903 | 7 | 855 | 6.05 | 19.11 |
| **83** | 3 | 1175 | 4 | 1005 | 5.15 | 12.85 |
| **84** | 0 | 1800 | 0 | 1800 | 3.93 | 4.09 |
| **85** | 0 | 1800 | 0 | 1800 | 4.22 | 4.76 |

| A005, 2 mg/kg group | | | | | | |
|---|---|---|---|---|---|---|
| **86** | 0 | 1800 | 0 | 1800 | 4.16 | 5.26 |
| **87** | 0 | 1800 | 0 | 1800 | 4.27 | 5.22 |
| **88** | 0 | 1800 | 1 | 1655 | 4.65 | 7.15 |
| **89** | 0 | 1800 | 0 | 1800 | 4.32 | 4.83 |
| **90** | 0 | 1800 | 0 | 1800 | 3.93 | 4.89 |

| A006, 0.125 mg/kg group | | | | | | |
|---|---|---|---|---|---|---|
| **91** | 0 | 1800 | 2 | 1453 | 3.93 | 4.11 |
| **92** | 1 | 1330 | 2 | 1325 | 3.88 | 4.21 |
| **93** | 4 | 877 | 7 | 707 | 5.98 | 16.58 |
| **94** | 5 | 798 | 8 | 758 | 5.79 | 15.19 |
| **95** | 4 | 808 | 7 | 738 | 5.18 | 17.07 |

| A006, 0.5 mg/kg group | | | | | | |
|---|---|---|---|---|---|---|
| **96** | 6 | 855 | 8 | 899 | 5.86 | 18.66 |
| **97** | 4 | 903 | 6 | 816 | 6.06 | 20.61 |
| **98** | 2 | 1175 | 3 | 1022 | 5.12 | 12.89 |
| **99** | 0 | 1800 | 0 | 1800 | 3.91 | 4.19 |
| **100** | 0 | 1800 | 0 | 1800 | 4.25 | 4.79 |

| A006, 2 mg/kg group | | | | | | |
|---|---|---|---|---|---|---|
| **101** | 0 | 1800 | 0 | 1800 | 4.66 | 5.13 |
| **102** | 0 | 1800 | 0 | 1800 | 4.16 | 5.28 |
| **103** | 0 | 1800 | 0 | 1800 | 4.65 | 4.97 |
| **104** | 0 | 1800 | 0 | 1800 | 4.67 | 4.79 |
| **105** | 0 | 1800 | 1 | 1660 | 3.98 | 7.87 |

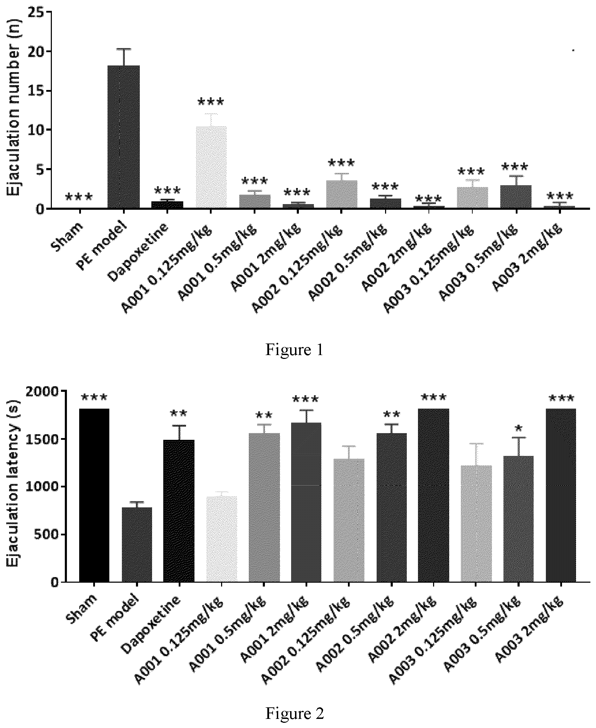

In this research, the number of ejaculations in Wistar rats was basically maintained at about 18 times within 30 min after PCA induction, and all groups showed a very significant decrease in the number of ejaculations compared with the PE model group (all achieved P<0.05). The average number of ejaculations in 30 min was 5 or less (see Figure 1). At a dose of 2 mg/kg, the efficacy of each of compounds A001 to A006 was better than dapoxetine hydrochloride.

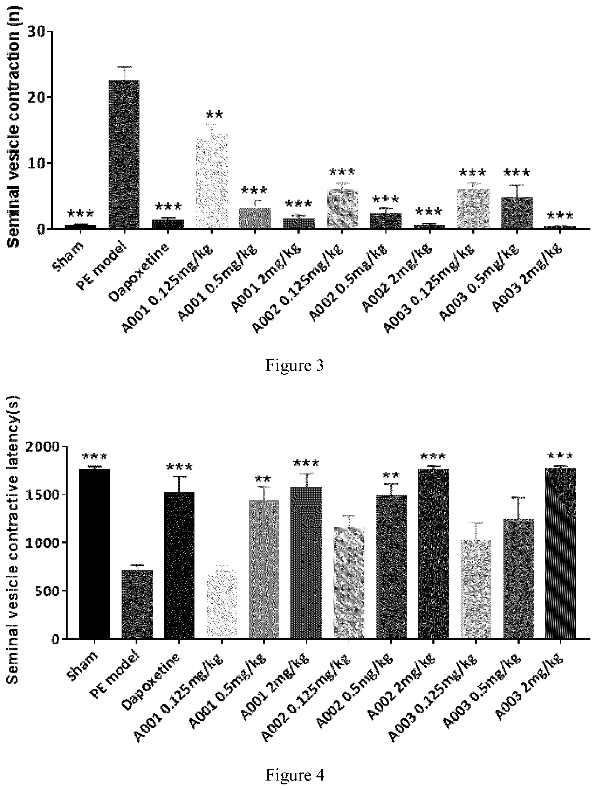

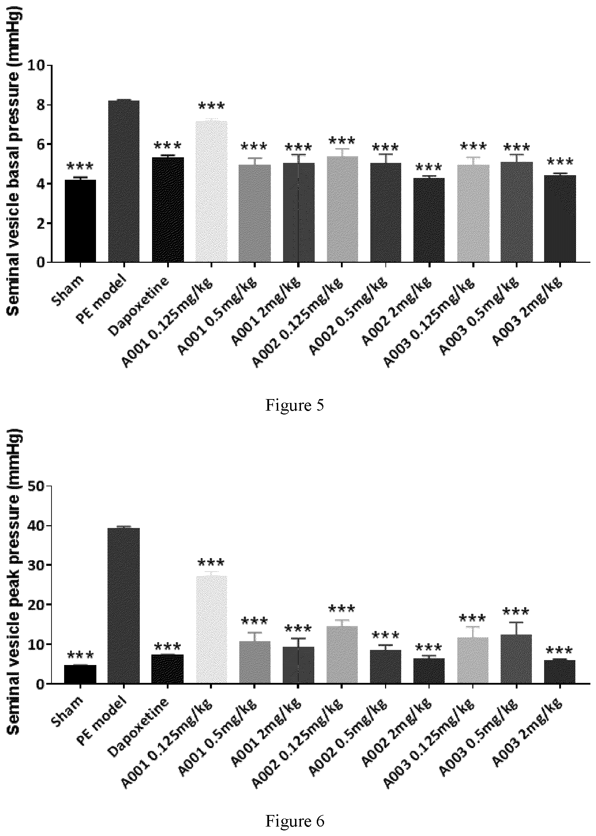

From the results of the initial ejaculation latency period, it can be seen that compared with the PE model group, each of the other groups showed a very significant extension in the initial ejaculation latency period (see Figure 2). In addition, within 30 min after PCA induction, the number of seminal vesicle contractions of the rats in the PE model group was about 22.4, while the number of seminal vesicle contractions in each of the other groups was less than 6, showing a very significant decrease in the number of seminal vesicle contractions compared with the PE model group (all achieved P<0.05) (see Figure 3). Moreover, from the observation of the latency period of seminal vesicle initial contraction in the rats, it can be seen that compared with the PE model group, Sham group, dapoxetine group, A001 medium dose group, A001 high dose group, A002 medium dose group, and A002 high dose group all showed a significant increase in the latency period of initial seminal vesicle contraction (P<0.001, P<0.001, P<0.01, P<0.001, respectively). The remaining groups (A001 low dose group and A002 low dose group) showed a delay to some extent but had no statistically significant differences compared with the PE model group (all of which P>0.05, see Figure 4). According to data from the monitoring of seminal vesicle basal pressure, all groups can effectively reduce the basal seminal vesicle pressure caused by PCA induction (all achieve P<0.001, see Figure 5). Similarly, data from the monitoring of seminal vesicle peak pressure indicated that all groups can very effectively reduce the seminal vesicle peak pressure caused by PCA induction (all achieve P<0.001) (see Figure 6). At an oral dose of 2 mg/kg, the efficacy of each of compounds A001 to A006 was better than that of dapoxetine hydrochloride administrated in 2 mg/kg by injection.

### 7.4 Conclusion

The potential effects of the tested drugs A001 to A006 and dapoxetine at different dose were monitored in PCA-induced Wistar rat premature ejaculation model. Statistical analysis of related indexes such as the number of ejaculations, the initial ejaculation latency period, seminal vesicle pressure, and cavernous body muscle contraction in rats revealed that dapoxetine and medium or high dose of A001 to A006 can effectively prolong the ejaculation latency period, and greatly reduce the number of ejaculations, reduce the seminal vesicle pressure and the number of the cavernous body muscle contraction. Compared with the efficacy of dapoxetine in treating PE, at a 2 mg/kg oral dose, the efficacy of A001 to A006 was better than that of dapoxetine hydrochloride administrated in 2 mg/kg by injection. In view of the bioavailability of 42% of dapoxetine hydrochloride as control, the compounds according to the present invention are significantly better than dapoxetine hydrochloride.

## Claims

1. A 2H-benzotriazole derivative or a pharmaceutically acceptable salt thereof, **characterized in that** the structure of the derivative is as shown by formula (I): 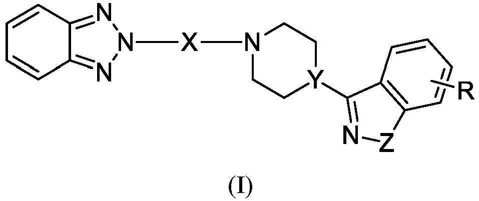 wherein
X represents: a C₃-C₄-alkyl or a C₃-C₄-alkoxy;
Y represents: CH or N;
Z represents: O or S;
R represents: optional substitution of hydrogen atom(s) by 1-3 halogen atoms selected from the group consisting of fluorine, chlorine, bromine.

2. The 2H-benzotriazole derivative or a pharmaceutically acceptable salt thereof according to claim 1, **characterized in that** the 2H-benzotriazole alkyl derivative is any one of the following compounds:
A001: 1-(4-(4-benzoisoxazolyl) piperazin-1-yl) butyl-2H-benzotriazole
A002: 1-(3-(4-(3-(6-fluorobenzoisoxazolyl)) piperidin-1-yl) propyl-2H-benzotriazole
A003: 1-(4-(4-(3-(6-fluorobenzoisoxazolyl)) piperidin-1-yl) butyl-2H-benzotriazole
A004: 1-(3-(4-benzisothiazolyl) piperazin-1-yl) propyl-2H-benzotriazole
A005: 1-(3-(4-(3-(6-fluorobenzoisoxazolyl)) piperazin-1-yl) propyl-2H-benzotriazole
A006: 1-(4-(4-(3-(6-fluorobenzoisoxazolyl)) piperazin-1-yl) butyl-2H-benzotriazole.

3. A pharmaceutical composition, **characterized in that** the pharmaceutical composition comprises any one or more of the 2H-benzotriazole derivatives or a pharmaceutically acceptable salt thereof according to claim 1 or 2, and also a pharmaceutically acceptable carrier.

4. The pharmaceutical composition according to claim 3, **characterized in that** the dosage form of the pharmaceutical composition is selected from tablets, sprays, oral soluble film agents, powders, capsules, and injections; and the content of active ingredient in the dosage form is 0.1% to 99.5%.

5. Use of the 2H-benzotriazole derivative or a pharmaceutically acceptable salt thereof according to claim 1 or 2 in the manufacture of medicaments for treating and/or preventing premature ejaculation.

6. Use of the pharmaceutical composition according to claim 3 or 4 in the manufacture of medicaments for treating and/or preventing premature ejaculation.

7. A method for preparing the 2H-benzotriazole derivative or a pharmaceutically acceptable salt thereof according to claim 1, **characterized in that** the method comprises the following synthetic scheme:
